(19) Europäisches Patentamt
European Patent Office
Office européen des brevets

(11) EP 1 787 648 A1

(12) EUROPEAN PATENT APPLICATION
published in accordance with Art. 158(3) EPC

(43) Date of publication:
**23.05.2007 Bulletin 2007/21**

(21) Application number: **05776962.2**

(22) Date of filing: **02.09.2005**

(51) Int Cl.:
***A61K 31/496*** (2006.01) ***A61K 9/06*** (2006.01)
***A61K 9/08*** (2006.01) ***A61K 9/10*** (2006.01)
***A61K 9/70*** (2006.01) ***A61P 25/18*** (2006.01)
***A61P 43/00*** (2006.01)

(86) International application number:
**PCT/JP2005/016084**

(87) International publication number:
**WO 2006/025516 (09.03.2006 Gazette 2006/10)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **03.09.2004 JP 2004257633**

(71) Applicant: **Dainippon Sumitomo Pharma Co., Ltd.
Osaka 541-8524 (JP)**

(72) Inventors:
- **MAEDA, Hiroo,
c/oDIANIPPON SUMITOMO PHARM, CO, LTD
Ibaraki-shi, Osaka 56 70878 (JP)**
- **OHARA, Naoki,
c/oDIANIPPON SUMITOMO PHARMA,CO,LTD.
Ibaraki-shi, Osaka 56 70878 (JP)**
- **IKEDA, Yuki,
c/o DAINIPPON SUMITOMO PHARMA CO,LTD
Ibaraki-shi, Osaka 56 70878 (JP)**

(74) Representative: **Vossius & Partner
Siebertstrasse 4
81675 München (DE)**

# (54) MEDICINAL COMPOSITION FOR PERCUTANEOUS PEROSPIRONE ADMINISTRATION

(57) A pharmaceutical composition for transdermal administration comprising perospirone of the formula (1):

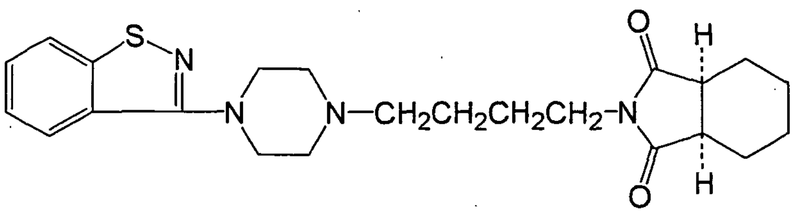

(1)

or a pharmaceutically acceptable acid addition salt thereof, which can inhibit the generation of metabolites and continuously maintain the blood level of perospirone.

EP 1 787 648 A1

## Description

TECHNICAL FIELD

The present invention relates to a pharmaceutical composition for transdermal administration comprising perospirone or a pharmaceutically acceptable acid addition salt thereof. In more detail, the present invention relates to a pharmaceutical composition for systemic transdermal administration whereby perospirone or a pharmaceutically acceptable acid addition salt thereof is transdermally absorbed and continuously delivered to a central tissue via a circulatory system.

BACKGROUND ART

A method for preparing perospirone (cis-N-[4-[4-(1,2-benzisothiazol-3-yl)-1-piperazinyl]butyl]cyclohexane-1,2-dicarboximide) of the formula (1) has been described in Patent Document 1.

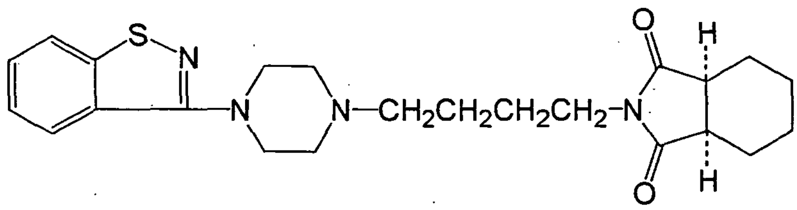

(1)

Perospirone or a pharmaceutically acceptable acid addition salt thereof, which is a serotonin-dopamine antagonist (SDA) which shows a strong binding activity and antagonistically acts to a dopamine 2 (D2) receptor and a serotonin 2 (5-HT2) receptor, and is useful as an antipsychotic agent.

However, perospirone was strongly metabolized via the first pass effect after oral administration (Nonpatent Document 1, 2 or 3). Additionally, it has been confirmed that among the main metabolic products ID-15036 of the formula (2) showed the strongest antiserotonin activity in rat, but the activity was about 1/8 compared to perospirone and any metabolites showed weak antidopamine activity in rat (Nonpatent Document 4).

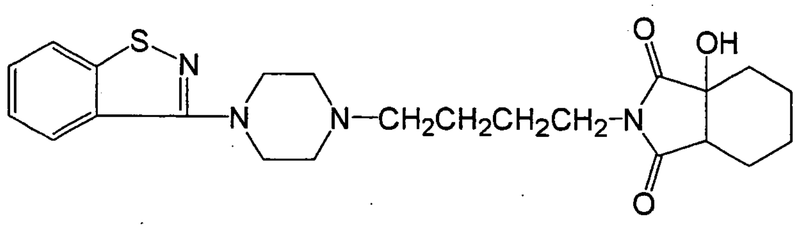

(2)

Therefore, there was a need of increasing an administering frequency in oral administration of the perospirone.

Recently, transdermal administration as an administration route has attracted attentions in a medical field because of an expectation to maintain drug levels in blood for a longer time and to extend biological half-lives compared to oral and intravenous injection administration, and further because of possibility of interruption of medication, easy administration and possible avoidance of the first pass effect in liver.

However, in case of applying drugs to transdermal administration, skin permeabilities of drugs are often barriers and the skin permeabilities depend largely on properties of drugs. In other words, some drugs are known not to be able to formulate transdermally because of their intrinsic properties. It has been known that the skin permeability of a drug (J) is defined as follows (Nonpatent Document 5). According to Fick's first law, it is represented by

$$J = A \cdot Cv \cdot K \cdot D / L$$

and a permeability coefficient (Kp) is represented by

$$Kp = K \cdot D/L$$

wherein A is an applied area of a transdermal formulation, Cv is a solubility of a drug, K is a distribution coefficient of a drug between the formulation and skin, D is a diffusion coefficient of a drug in skin, and L is an effective skin thickness, and from the above two equations it is defined as follows:

$$J = A \cdot Cv \cdot Kp \text{ (Equation 1)}$$

Because Kp can be predicted by a molecular weight and a distribution coefficient which are drug properties in the manner of a report of Guy et al. (Nonpatent Document 6), the amount of drug permeation per unit area of skin applying a formulation can be calculated by assigning predictive values for perospirone Kp, Cv (a solubility of a drug) and A (1 $cm^2$) to Equation 1. The amount of permeation of perospirone per unit area was calculated in this way to give 0.71 $\mu$g/day/$cm^2$. For example, in case of applying formulations in 25 $cm^2$, the amount of perospirone which permeated via skin would be 18 $\mu$g/day, and the amount of perospirone as a transdermal formulation needed to permeate via skin would be estimated to be more than 120 $\mu$g/day. Therefore, it was thought to be extremely difficult to absorb the desired amount of perospirone via skin with estimation by the above Equation 1 based on drug properties. In other words, it was thought that it would be impossible to transdermally administer perospirone according to the above estimation based on the drug properties.

On the other hand, a transdermal formulation of tandospirone (for example, Patent Document 2) is known as a pharmaceutical composition for transdermal administration of a central nervous system drug, but the diseases to be treated thereby are psychosomatic disease, depression in nervous diseases or fear, which are different from schizophrenia of the disease to be treated by perospirone.

Patent Document 1: JP-A-62-123179
Patent Document 2 : JP-A-11-228414
Nonpatent Document 1: KISO TO RINSHO (Basis and Clinic) 31, 543-568, 1997
Nonpatent Document 2 : KISO TO RINSHO (Basis and Clinic) 31, 737-753, 1997
Nonpatent Document 3: KISO TO RINSHO (Basis and Clinic) 31, 2113-2157, 1997
Nonpatent Document 4: KISO TO RINSHO (Basis and Clinic) 31, 893-902, 1997
Nonpatent Document 5 : IYAKUHIN NO KAIHATU (Development of Medicine) 13, 87-133, 1989
Nonpatent Document 6: American Journal of Industrial Medicine 23, 711-719, 1993

DISCLOSURE OF INVENTION

A problem to be resolved by the present invention is to provide a method for administering perospirone or a pharmaceutically acceptable acid addition salt thereof which can inhibit a generation of a metabolite and continuously maintain a blood level thereof.

According to extensive studies to solve the above problem, the inventors have surprisingly found that a pharmaceutical composition for transdermal administration comprising perospirone or a pharmaceutically acceptable acid addition salt thereof can give a high blood level of the perospirone, i.e. the perospirone can penetrate through skin via the pharmaceutical composition for transdermal administration of the present invention, that a generation of a metabolite of perospirone can be remarkably reduced by the pharmaceutical composition for transdermal administration of the present invention, and that it can continuously maintain the blood level to exhibit continuously the efficacy of perospirone, and have achieved the present invention, although it has been considered that it will be difficult to transdermally administer perospirone because of the very low skin permeability thereof by assumption in the light of its properties as mentioned above.

Thus, the present invention provides the following embodiments.

(1) A pharmaceutical composition for transdermal administration comprising perospirone of the formula (1):

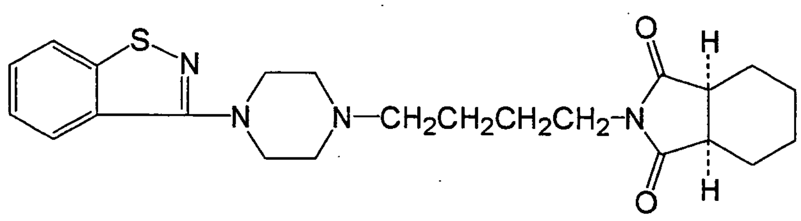

(1)

or a pharmaceutically acceptable acid addition salt thereof.

(2) A pharmaceutical composition for transdermal administration of the above (1) comprising perospirone hydrochloride.

(3) A pharmaceutical composition for systemic transdermal administration comprising perospirone or a pharmaceutically acceptable acid addition salt thereof as an active ingredient.

(4) A pharmaceutical composition for systemic transdermal administration of the above (3) comprising perospirone hydrochloride as an active ingredient.

(5) A pharmaceutical composition for systemic transdermal administration of the above (3) or (4) in the form of a tape, a patch, a cataplasm, an ointment, a cream, a lotion, a liquid or a gel.

(6) Use of perospirone or a pharmaceutically acceptable acid addition salt thereof for preparing a pharmaceutical composition for systemic transdermal administration of the above (3) or (4).

(7) A pharmaceutical composition for transdermal administration of the above (1) or (2) for treating schizophrenia.

(8) A pharmaceutical composition for systemic transdermal administration of the above (3), (4) or (5) for treating schizophrenia.

The present invention can provide a pharmaceutical composition for transdermal administration comprising perospirone or a pharmaceutically acceptable acid addition salt thereof which can remarkably reduce the generation of a metabolite and can continuously maintain a blood level thereof.

BRIEF DESCRIPTION OF DRAWINGS

Figure 1: A graph of serum concentration of perospirone and the main metabolite ID-15036 4 hours after transdermal administration of Formulations 1 to 8 prepared in Examples 1 to 8 (Experiment 1), and 1 hour after oral administration of an aqueous solution of perospirone hydrochloride in Comparative Experiment 1.

Figure 2: A graph of a temporal shift of perospirone and the main metabolite ID-15036 in blood in case of transdermal administration of Formulation 1 prepared in Example 1.

Figure 3: A graph of a temporal shift of perospirone and the main metabolite ID-15036 in blood in case of transdermal administration of Formulation 4 prepared in Example 4.

Figure 4: A graph of a temporal shift of perospirone and the main metabolite ID-15036 in blood in case of transdermal administration of Formulation 5 prepared in Example 5.

Figure 5: A graph of a temporal shift of perospirone and the main metabolite ID-15036 in blood in case of oral administration of perospirone hydrochloride hydrate in Comparative Experiment 1.

Figure 6: A graph of a temporal shift of perospirone in blood in case of transdermal administration of Formulation 11 prepared in Example 15.

BEST MODE FOR CARRYING OUT THE INVENTION

A pharmaceutically acceptable acid addition salt of perospirone includes, for example, an inorganic acid addition salt such as hydrochloride, hydrobromide, sulfate, phosphate or nitrate, or an organic acid addition salt such as acetate, propionate, succinate, lactate, malate, tartrate, citrate, maleate, fumarate, methanesulfonate, p-toluenesulfonate or ascorbate.

Additionally, the present invention also includes a hydrate or a solvate (e.g. ethanolic solvate) of perospirone or a pharmaceutically acceptable acid addition salt thereof.

A formulation form in "a pharmaceutical composition for transdermal administration comprising perospirone or a pharmaceutically acceptable acid addition salt thereof" of the present invention includes any conventional dosage form for external preparation, such as a tape, a patch, a cataplasm, an ointment, a cream, a lotion, a liquid, a gel or the like, preferably a tape or a patch.

The external preparations in these dosage forms can be prepared by usual methods using a conventional adhesive, base or the like. For example, it can be prepared according to the methods described in "Keihi Tekiyou Seizai Kaihatu Manual (A Development Manual of Formulations for Transdermal Application)" edited by Mitsuo MATSUMOTO (1985), JP-2651616, WO96/12465, JP-A-9-278651.

An adhesive for a tape or a patch includes, for example, an acrylic adhesive, a rubber adhesive, a silicon adhesive or the like.

An acrylic adhesive includes, for example, a (co)polymer of alkyl (meta)acrylate (as a main component). The (co)polymer may be either a copolymer of two or more kinds of alkyl (meta)acrylate or a copolymer of an alkyl (meta) acrylate with a functional monomer which is copolymerizable with alkyl (meta)acrylate. The alkyl acrylate includes, for example, acrylates which are esterified by a straight chain or branched chain alkyl having 1 to 18 carbon atoms, specifically methyl (meta)acrylate, butyl (meta)acrylate, hexyl (meta)acrylate, octyl (meta)acrylate, nonyl (meta)acrylate, decyl (meta) acrylate or the like. The functional monomer includes, for example, a monomer having a hydroxyl group (e.g. hydroxyethyl (meta)acrylate), a monomer having a carboxyl group (e.g. butyl maleate or crotonic acid), a monomer having an amide group (e.g. (meta)acrylamide), a monomer having an amino group (e.g. dimethylaminoacrylic acid esters), a monomer having a pyrrolidone ring (e.g. N-vinyl-2-pyrrolidone), or the like.

The rubber adhesive includes, for example, a natural rubber, a polyisopropylene rubber, a polyisobutylene rubber, a styrene-isoprene-styrene block copolymer or a styrene-butadiene-styrene block copolymer, wherein the main component is a rubber elastomer.

The silicon adhesive includes, for example, a polydimethylsiloxane or a diphenylsiloxane wherein the main component is a silicon rubber.

The perospirone or a pharmaceutically acceptable acid addition salt thereof can be dispersed in these adhesives to give a "microreserver-type" formulation, or a solution of perospirone or a pharmaceutically acceptable acid addition salt thereof can be filled therein to give a "reserver-type" formulation.

A base for the ointment or the cream preparation includes, for example, a fatty oil, a lanolin, a petrolatum, a paraffin, a plastibase, glycols, a higher fatty acid, a higher alcohol or the like. A stabilizing agent, an antiseptic agent, an emulsifying agent, a suspending agent or the like may be optionally added to the base.

A base for the lotion includes, for example, ethanol, glycerine, glycol or the like.

A base for the liquid preparation includes, for example, ethanol, water, glycol or the like.

A base for the gel preparation includes, for example, a product obtained by gelatinizing liquid fats and oils by a gelling agent for an oily gel preparation, or a gelling agent such as a carboxymethyl polymer, a hydroxypropylcellulose or a polyvinyl alcohol for an aqueous gel preparation.

In the "reserver-type" formulation or the lotion, the solvent for dissolving perospirone or a pharmaceutically acceptable acid addition salt thereof includes, for example, an organic solvent, a buffer solution or a mixed solvent of the organic solvent with water or the buffer solution which can dissolve perospirone or a pharmaceutically acceptable acid addition salt thereof and have a low skin irritation. Additionally, pH of the buffer solution is preferably neutral in view of the skin irritancy.

The pharmaceutical composition for transdermal administration of the present invention may optionally further comprise various pharmaceutically acceptable additives, such as stabilizing agents, antioxidants, flavoring agents, bulking agents or other transdermal absorption enhancers, unless they give any bad effect to the object of the present invention. Additionally, pH is preferably weakly basic in view of a skin permeability.

An especially preferable one among the dosage forms is a tape, a patch or the like which can control perospirone blood level at a constant level.

An additive which is added to a tape or a patch includes α-terpineol, isopropyl myristate, 1-menthol, lauric acid, lauryl alcohol, crotamiton, diethyl sebacate, N-methyl-2-pyrrolidone, Azone (Registered Trademark) or the like.

A dose may change depending on conditions of patients such as ages or weights, symptoms, forms of formulations, and it is usually administered as perospirone in an amount of 0.01 mg to 1.0 g/day in adults.

Perospirone blood level to be maintained may be adjusted depending on conditions of patients, but it is preferable within the maximum blood level which has been shown by oral administration of perospirone hydrochloride dihydrate in the current clinical use in view of side-effects.

According to the present invention, an effective perospirone blood level, an antidopaminergic activity and an antiserotonin activity can be continued for 5 hours to 4 weeks, preferably for 1 day to 1 week. Further, a long-term-antipsychotic effect can be achieved by removing used formulations and newly using the formulation of the present invention.

The present invention is explained in more detail by the following Examples, but is not intended to be limited to these examples in any way.

Example 1

Preparation of Perospirone Tape

A styrene-isoprene-styrene block copolymer (Quintac 3421) (2.00 g), a liquid paraffin (3.00 g), a polybutene (NISSEKI Polybutene HV-300) (1.50 g) and an alicyclic saturated hydrocarbon resin (Alcon P-100) (2.50 g) were dissolved in hexane to prepare an adhesive solution. Thereto was added perospirone so that its content in a matrix layer was 10%, and the mixture was stirred thoroughly. Then, the mixture was spread onto a support in a thickness of about 100 μm, dried, pasted with a release liner and cut in a size of 4 cm x 4 cm to prepare perospirone tape (Formulation 1).

Example 2

Preparation of Perospirone Hydrochloride Tape

A styrene-isoprene-styrene block copolymer (Quintac 3421) (2.00 g), a liquid paraffin (3.00 g), a polybutene (NISSEKI polybutene HV-300) (1.50 g) and an alicyclic saturated hydrocarbon resin (Alcon P-100) (2.50 g) were dissolved in hexane to prepare an adhesive solution. Thereto was added perospirone hydrochloride so that its content in a matrix layer was 10%, and the mixture was stirred thoroughly. Then, the mixture was spread onto a support in a thickness of about 100 μm, dried, pasted with a release liner and cut in a size of 4 cm x 4 cm to prepare perospirone hydrochloride tape (Formulation 2).

Example 3

Preparation of Perospirone Aqueous Ointment

Perospirone (0.15 g) was added to macrogol 400 (NOF Corporation) (1.425 g) to be dissolved and the mixture was warmed to 70°C. This solution (1.05 g) was mixed with macrogol 4000 (NOF Corporation) (0.95 g) dissolved at 70°C to prepare perospirone aqueous ointment (Formulation 3).

Example 4

Preparation of Perospirone Hydrochloride Aqueous Ointment

Macrogol 4000 (NOF Corporation) and macrogol 400H (NOF Corporation) were weighed 50 g each to be dissolved at 70°C and mixed homogeneously. Then, the mixture was cooled gradually with stirring. The mixture (9.5 g) and perospirone hydrchloride hydrate (0.5 g) were combined together to prepare perospirone hydrochloride aqueous ointment (Formulation 4).

Example 5

Preparation of Perospirone Oily Ointment

Perospirone (0.5 g) and plastibase 50W (Registered Trademark: Bristol-Myer Co.) (9.5 g) were combined together to prepare perospirone oily ointment (Formulation 5).

Example 6

Preparation of Perospirone Hydrochloride Oily Ointment

Perospirone hydrochloride hydrate (0.5 g) and plastibase 50W (Registered Trademark: Bristol-Myer Co.) (9.5 g) were combined together to prepare perospirone hydrochloride oily ointment (Formulation 6).

Example 7

Preparation of Perospirone Solution

Perospirone (0.7 g) was dissolved in a phosphate buffer (70 ml) comprising 50% ethanol to prepare perospirone solution (Formulation 7).

Example 8

Preparation of Perospirone Hydrochloride Solution

Perospirone hydrochloride (0.7 g) was dissolved in a phosphate buffer (70 ml) comprising 50% ethanol to prepare perospirone hydrochloride solution (Formulation 8).

Example 9

Preparation of Perospirone Tape

A styrene-isoprene-styrene block copolymer (Quintac 3421) (2.00 g), a liquid paraffin (3.00 g), a polybutene (NISSEKI polybutene HV-300) (1.50 g), an alicyclic saturated hydrocarbon resin (Alcon P-100) (2.50 g) and L-menthol (0.4 g) were dissolved in hexane to prepare an adhesive solution. Thereto was added perospirone so that its content in a matrix layer was 10%, and the mixture was stirred thoroughly. Then, the mixture was spread onto a support in a thickness of about 100 μm, dried, pasted with a release liner and cut in a size of 4 cm x 4 cm to prepare perospirone tape (Formulation 9).

Example 10

Preparation of Perospirone Tape

A styrene-isoprene-styrene block copolymer (Quintac 3450) (17 g), a polybutene (NISSEKI polybutene HV-300) (15 g), an alicyclic saturated hydrocarbon resin (Alcon P-100) (35 g) and a liquid paraffin (25 g) are heated to be dissolved. Thereto are added a crotamiton (3 g) and perospirone (5 g), and the mixture is mixed homogenously. The mixture is spread onto a support, covered with a liner, and cut in a size of 4 cm x 4 cm to prepare perospirone tape.

Example 11

Preparation of Perospirone Tape

A polyisobutylene (Oppanol B150) (50 g), a polybutene (NISSEKI polybutene HV-300) (30 g) and an alicyclic saturated hydrocarbon resin (Alcon P-100) (20 g) are dissolved in hexane. Thereto is added perospirone so that its content in a matrix layer is 10%, and the mixture is stirred thoroughly. Then, the mixture is spread onto a support in a thickness of about 20 μm, dried at room temperature for 1 week, pasted with a release liner and cut in a size of 4 cm x 4 cm to prepare perospirone tape.

Example 12

Preparation of Perospirone Tape

An acrylic adhesive (Oribain BPS4849-40) (15 g) and a curative agent (BHS8515) (0.41 g) are combined together. Thereto is added perospirone so that its content in a matrix layer is 10%, and the mixture is stirred thoroughly. Then, the mixture is spread onto a support in a thickness of about 20 μm, dried at room temperature for 1 week, pasted with a release liner and cut in a size of 4 cm x 4 cm to prepare perospirone tape.

Example 13

Preparation of Perospirone Ointment

Perospirone ointment is prepared from perospirone (6 g), a carboxyvinyl polymer (2 g), triethanolamine (2 g), ethanol (50 g) and water (40 g).

Example 14

Preparation of Perospirone Tape

A styrene-isoprene-styrene block copolymer (Quintac 3421) (8 g), a liquid paraffin (12 g), a polybutene (NISSEKI

polybutene HV-300) (6 g) and an alicyclic saturated hydrocarbon resin (Alcon P-100) (10 g) were dissolved in ethyl acetate (20 ml) to prepare an adhesive solution. Thereto was added perospirone (4 g), and the mixture was stirred thoroughly at 45°C. Then, the mixture was spread onto a polyethylene terephthalate film in a thickness of about 270 μm, dried and pasted with a release liner to prepare perospirone tape (Formulation 10).

Example 15

Preparation of Perospirone Tape

A polyisobutylene (Oppanol B100) (1 g), a polybutene (NISSEKI polybutene HV300) (0.75 g), an alicyclic saturated hydrocarbon resin (Alcon P-100) (1.25 g), a liquid paraffin (1.5 g) and perospirone (0.5 g) were dissolved in hexane (20 ml) and ethyl acetate (2 ml). The mixture was stirred thoroughly and defoamed. Then, the mixture was spread onto a polyethylene terephthalate film in a thickness of about 400 μm, dried at room temperature and pasted with a release liner to prepare perospirone tape (Formulation 11).

Example 16

Preparation of Perospirone Tape

A polyisobutylene (Oppanol B100) (1.25 g), a polybutene (NISSEKI polybutene HV300) (0.5 g), an alicyclic saturated hydrocarbon resin (Alcon P-100) (0.5 g), a liquid paraffin (0.25 g), a isopropyl myristate (1.75 g) and perospirone (0.75 g) were dissolved in hexane (20 ml) and ethyl acetate (2 ml). The mixture was stirred thoroughly and defoamed. Then, the mixture was spread onto a polyethylene terephthalate film in a thickness of about 400 μm, dried at room temperature and pasted with a release liner to prepare perospirone tape (Formulation 12).

Reference Example 1

Preparation of Placebo Tape

A styrene-isoprene-styrene block copolymer (Quintac 3421) (8 g), a liquid paraffin (12 g), a polybutene (NISSEKI polybutene HV-300) (6 g) and an alicyclic saturated hydrocarbon resin (Alcon P-100) (10 g) were dissolved in ethyl acetate (20 ml) to prepare an adhesive solution. This solution was stirred thoroughly at 45°C, and then spread onto a polyethylene terephthalate film in a thickness of about 270 μm, dried and pasted with a release liner to prepare a placebo tape (Reference Formulation 1).

Experiment 1

Efficacy (Antiserotonin Activities) of Transdermal Absorption Formulations

Antiserotonin activities of the perospirone tape prepared in Example 14 (Formulation 10) and the perospirone tape prepared in Example 15 (Formulation 11) were assessed in a rat tryptamine-induced clonic seizures of forepaws model. The test was carried out according to the description of Jpn.J.Pharmacol. 53, 321-329 (1990).
Specifically, 7-week-old SD rats were dehaired the back on the day before the test, the tapes were patched on the back, and further water was administered orally in an amount of 5 ml/Kg. After the patching of tapes, a tryptamine hydrochloride was administered via tail veins in a dose of 40 mg/Kg at predetermined times as assessment times, and appearance of a clonic seizures of forepaws was observed. When no clonic seizures was observed for five minutes after an administration of a tryptamine hydrochloride, an inhibitory effect of a tryptamine-induced behavior was determined to be positive.
Patched perospirone tapes and assessment times, and population used in each case and assessment results are shown in Table 1.

Comparative Experiment 1

Efficacy (Antiserotonin Activities) of Oral Administration

7-Week-old SD rats were dehaired the back on the day before the test, patched with the placebo tape (Reference Formulation 1) containing no perospirone prepared in Reference Example 1 on the back, and administered orally perospirone hydrochloride hydrate in a dose of 3 mg/Kg or 0.5 mg/Kg. After administrations, a tryptamine hydrochloride was administered via tail veins in a dose of 40 mg/Kg at predetermined times as assessment times. An inhibitory effect

of a tryptamine-induced behavior was determined in the same manner as Experiment 1. The assessment results are shown in Table 1.

Table 1

| Antiserotonin Activities in Tryptamine-Induced Clonic Seizures of Forepaws Model | | | | | |
|---|---|---|---|---|---|
| | Transdermal Formulation/ Patch Area | Oral Administration | Efficacy (positive numbers*/ total numbers) | | |
| | | | 1 hour | 10 hours | 48 hours |
| Expt. 1 | Formulation 10/<br>1 cm x 1 cm<br>0.5 cm x 0.5 cm | <br>water<br>water | <br>5/5<br>5/5 | <br>7/7<br>7/7 | <br>6/6<br>4/5 |
| | Formulation 11/<br>1 cm x 1 cm | <br>water | <br>- | <br>- | <br>5/6 |
| Comp. Expt. 1 | Reference<br>Formulation 1/ 1 cm x 1 cm | 3 mg/Kg<br>0.5 mg/Kg | 3/5<br>0/5 | 0/7<br>- | -<br>- |
| *: The number which showed a positive inhibitory effect of a tryptamine-induced behavior<br>-: Not Determined. | | | | | |

From the results of Experiment 1 and Comparative Experiment 1 shown in Table 1, antiserotonin activities could not be recognized 10 hours after administration by oral administration of perospirone to the model even in a group of 3 mg/Kg where the efficacy could be recognized 1 hour after administration. On the other hand, the perospirone tapes prepared in Example 14 and Example 15 (Formulation 10, 11) showed antiserotonin activities either 1, 10 or 48 hours after patching. The results show that perospirone transdermal formulation had a remarkable longstanding drug efficacy.

Experiment 2

Efficacy (Antidopaminergic Activities) of Transdermal Formulations

Antidopaminergic activities of the perospirone tape prepared in Example 14 (Formulation 10) and the perospirone tape prepared in Example 15 (Formulation 11) were assessed in a rat apomorphine-induced stereotypy model. The test was carried out according to the description of Jpn.J.Pharmacol. 53, 321-329 (1990).

Specifically, 7-week-old SD rats were dehaired the back on the day before the test, patched the tapes on the back, and further water was administered orally in an amount 5 ml/Kg. After the patching, an apomorphine hydrochloride was administered via tail veins in a dose of 1.25 mg/Kg at predetermined times as assessment times, and stereotypy was observed. When no action of licking or biting was observed for thirty minutes after an administration of an apomorphine hydrochloride, an inhibitory effect of an apomorphine hydrochloride-induced behavior was determined to be positive.

Patched perospirone tapes and assessment times, and population used in each case and assessment results are shown in Table 2.

Comparative Experiment 2

Efficacy (Antidopaminergic Activities) of Oral Formulations

7-Week-old SD rats were dehaired the back on the day before the test, patched the placebo tape (Reference Formulation 1) containing no perospirone prepared in Reference Example 1 on the back, and administered orally perospirone hydrochloride hydrate in a dose of 10 mg/Kg or 2 mg/Kg. Thereafter, an apomorphine hydrochloride was administered via tail veins in a dose of 1.25 mg/Kg at predetermined times as assessment times. An inhibitory effect of an apomorphine-induced behavior was determined in the same manner as Experiment 2. The assessment results are shown in Table 2.

Table 2

| Antidopaminergic Activities in Apomorphine-Induced Stereotypy Model | | | | | |
|---|---|---|---|---|---|
| | Transdermal Formulation/ Patch Area | Oral Administration | Efficacy (positive numbers*/ total numbers) | | |
| | | | 1 hour | 10 hours | 48 hours |
| Expt. 2 | Formulation 10/<br>1 cm x 1 cm<br>0.5 cm x 0.5 cm | <br>water<br>water | <br>5/5<br>3/5 | <br>5/5<br>5/5 | <br>5/5<br>5/5 |
| | Formulation 11/<br>1 cm x 1 cm<br>0.5 cm x 0.5 cm | <br>water<br>water | <br>-<br>- | <br>5/5<br>5/5 | <br>5/5<br>- |
| Comp. Expt. 2 | Reference Formulation 1/<br>1 cm x 1 cm | 10 mg/Kg<br>2 mg/Kg | 5/5<br>2/5 | 4/5<br>- | 0/5<br>- |
| *: The number which showed a positive inhibitory effect of an apomorphine-induced behavior<br>-: Not Determined | | | | | |

From the results of Experiment 2 and Comparative Experiment 2 shown in Table 2, a group orally administered with perospirone showed antidopaminergic activities 1 and 10 hours after administration but not 48 hours after administration in said model. On the other hand, the perospirone tapes prepared in Example 14 and Example 15 (Formulation 10, 11) showed antidopaminergic activities either 1, 10 or 48 hours after patching. The results show that perospirone transdermal formulation had a remarkable longstanding drug efficacy.

Experiment 3

Blood Kinetics Assessment of Transdermal Formulations

7-Week-old SD male rats were anesthetized and dehaired the abdomen. The rats were administered with transdermal formulations Formulations 1 to 8 in the sites of the dehaired abdomen and constantly collected blood, and serums were separated. The serums were partitioned and extracted with n-hexane-chloroform (7:3) and about 0.0005M aqueous sodium hydroxide solution, and the organic layers were evaporated and dried to solids: The resulting solids were dissolved, and serum concentrations of perospirone and its main metabolite ID-15036 were determined by quantification via reverse phase high performance liquid chromatography. Application of the formulation was carried out, in case of the tapes (Formulations 1, 2) by patching in a size of 4 cm x 4. cm (n=3); in case of the ointments (Formulations 3 to 6) by coating 0.9 g of the formulations in a range of 5 cm x 6 cm (n=2); in case of the solutions (Formulations 7, 8) by fixing cylindrical glass cells on rats' abdomens via biomedical adhesives and injecting the formulations (10 mL) into the glass cells (n=3).

Comparative Experiment 3

Blood Kinetics Assessment in Oral Administration

7-Week-old SD male rats were administered orally with 10 mg/kg of a solution of perospirone hydrochloride hydrate in a distilled water for injection in a concentration of 2 mg/ml, and the blood was collected with lapse of time. Serum concentrations of perospirone and the main metabolite ID-15036 were determined in the same manner as Experiment 3.

Serum concentration of perospirone and the main metabolite ID-15036 4 hours after administration of Formulations 1 to 8 used in Experiment 3 and 1 hour after administration of Comparative Experiment 3 (oral administration of perospirone hydrochloride) are shown in Figure 1. Also, concentration ratios of ID-15036 to perospirone (ID-15036/perospirone) are shown in Table 3 for formulations when ID-15036 could be measured at a concentration above lower limit for quantitation in this experiment. As is clear from Figure 1 and Table 3, it has been confirmed that in oral administration a metabolism of perospirone was so fast that a serum concentration of a metabolite of perospirone ID-15036 was about 4 times higher than that of perospirone, while in transdermal administration of the present invention, on the contrary to oral administration, serum concentrations of perospirone was 7 to 19 times higher than those of ID-15036.

Table 3

| ID-15036/Perospirone Ratio in Serum after Transdermal Administration | |
|---|---|
| | ID-15036/Perospirone Ratio in Serum |
| Formulation 1 (4 hours after transdermal administration) | 0.063 |
| Formulation 3 (4 hours after transdermal administration) | 0.143 |
| Formulation 5 (4 hours after transdermal administration) | 0.095 |
| Formulation 7 (4 hours after transdermal administration) | 0.052 |
| Formulation 8 (4 hours after transdermal administration) | 0.056 |
| Comp. Expt. 3 (1 hour after oral administration) | 4.17 |
| Comp. EXPT. 3 (4 hours after oral administration) | 3.75 |

Additionally, temporal shifts of perospirone and the main metabolite ID-15036 in blood in formulations 1, 4, 5 and Comparative Experiment 3 (oral administration of perospirone hydrochloride) are shown in Figures 2, 3, 4 and Figure 5, respectively. From the results of blood kinetics assessments of transdermal absorption formulations in Experiment 3 (Figure 2, 3, 4), and blood kinetics test in oral administration of Comparative Experiment 3 (Figure 5), in oral administration (Figure 5) maximum serum concentrations were observed 30 minutes after administration of the first measurement point, and serum concentrations were rapidly reduced thereafter, while transdermal absorption formulations of the present invention (Figures 2, 3, 4) showed maximum serum concentrations 4 to 8 hours after administration and maintained high perospirone concentrations without rapidly reducing serum concentrations thereafter.
These results show that a pharmaceutical composition for transdermal administration of perospirone or perospirone hydrochloride of the present invention has an effect of maintaining perospirone concentrations in blood for a long term, and suppressing metabolite (ID-15036) concentrations to a large extent compared to oral administration. Experiment 4

Blood Kinetics Assessment of Transdermal Absorption Formulations

7-Week-old SD male rats were anesthetized and dehaired the back. The rats were administered with Formulation 11 cut in a size of 1.5 cm x 1.5 cm at the site of the dehaired back (n=4) and the blood was collected with the lapse of time, and serum was separated therefrom. The serum was partitioned and extracted with n-hexane-chloroform (7:3) and about 0.0005M aqueous sodium hydroxide solution, and the organic layers were evaporated and dried to solids. The resulting solids were dissolved, and serum concentration of perospirone was determined by reverse phase high performance liquid chromatography.
Serum concentration of perospirone is shown in Figure 6. As is clear from Figure 6, the pharmaceutical composition for transdermal administration of perospirone of the present invention has an effect of maintaining perospirone concentration in blood for a long term by the days.

INDUSTRIAL APPLICABILITY

A pharmaceutical composition for transdermal administration comprising perospirone or a pharmaceutically acceptable acid addition salt thereof of the present invention can be remarkably reduce the generation of metabolites and continuously maintain the blood level of perospirone, and hence can be used as a therapeutic agent.

**Claims**

1. A pharmaceutical composition for transdermal administration comprising perospirone of the formula (1):

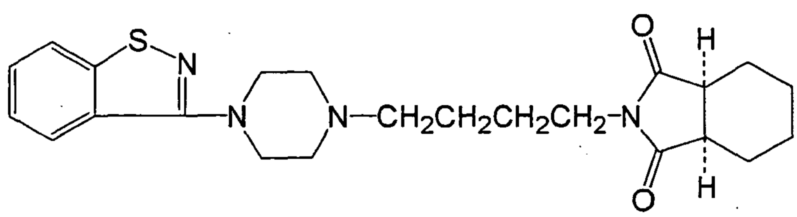

(1)

or a pharmaceutically acceptable acid addition salt thereof.

2. The pharmaceutical composition for transdermal administration of claim 1 comprising perospirone hydrochloride.

3. A pharmaceutical composition for systemic transdermal administration comprising as an active ingredient perospirone or a pharmaceutically acceptable acid addition salt thereof.

4. The pharmaceutical composition for systemic transdermal administration of claim 3 comprising as an active ingredient perospirone hydrochloride.

5. The pharmaceutical composition for systemic transdermal administration of claim 3 or 4, which is in the form of a tape, a patch, a cataplasm, an ointment, a cream, a lotion, a liquid or a gel.

6. Use of perospirone or a pharmaceutically acceptable acid addition salt thereof for preparing the pharmaceutical composition for systemic transdermal administration as set forth in claim 3 or 4.

7. The pharmaceutical composition for transdermal administration of claim 1 or 2 for treating schizophrenia.

8. The pharmaceutical composition for systemic transdermal administration of claim 3, 4 or 5 for treating schizophrenia.

Figure 1

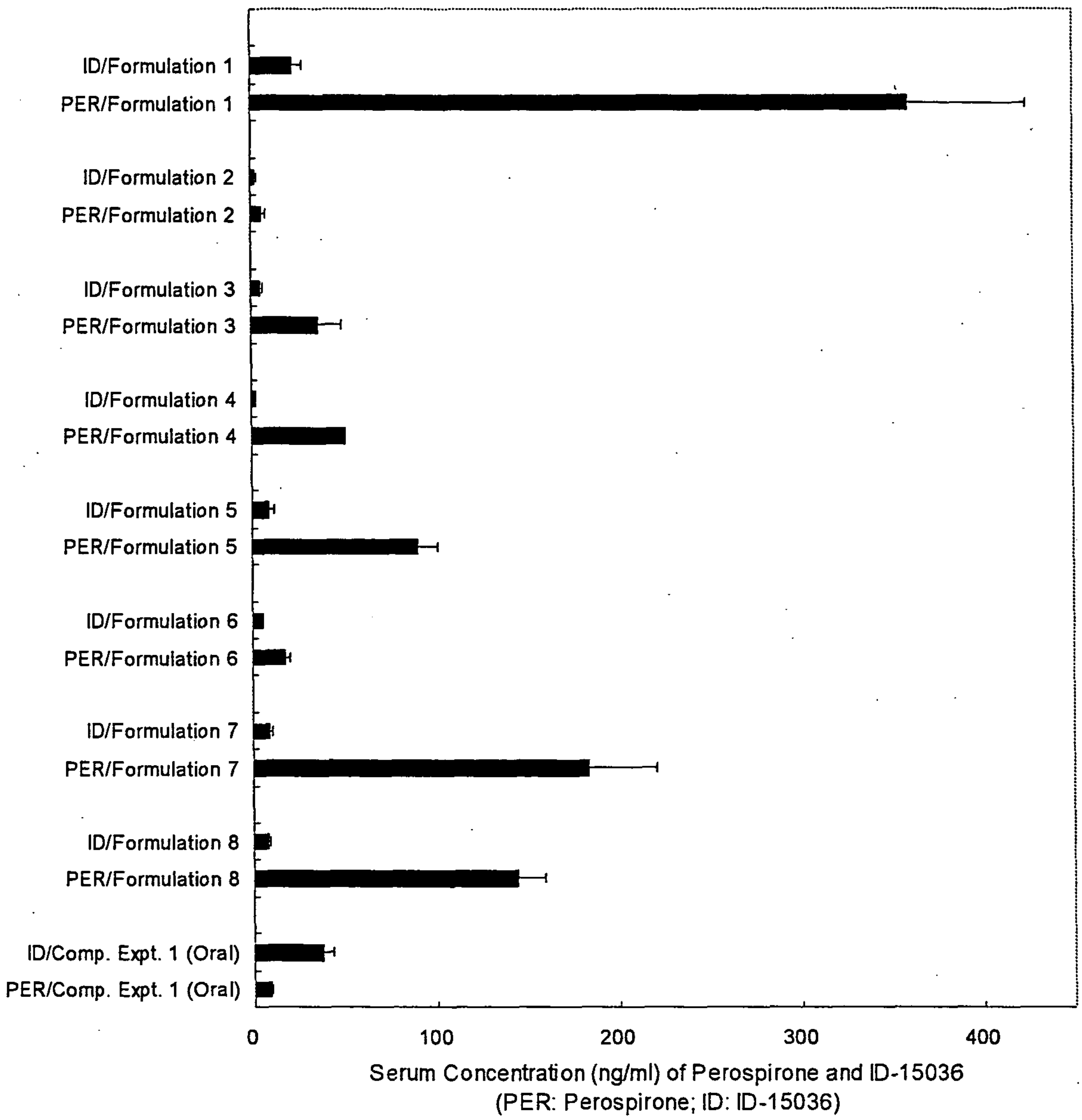
ID/Formulation 1
PER/Formulation 1
ID/Formulation 2
PER/Formulation 2
ID/Formulation 3
PER/Formulation 3
ID/Formulation 4
PER/Formulation 4
ID/Formulation 5
PER/Formulation 5
ID/Formulation 6
PER/Formulation 6
ID/Formulation 7
PER/Formulation 7
ID/Formulation 8
PER/Formulation 8
ID/Comp. Expt. 1 (Oral)
PER/Comp. Expt. 1 (Oral)
0
100
200
300
400
Serum Concentration (ng/ml) of Perospirone and ID-15036
(PER: Perospirone; ID: ID-15036)

Figure 2

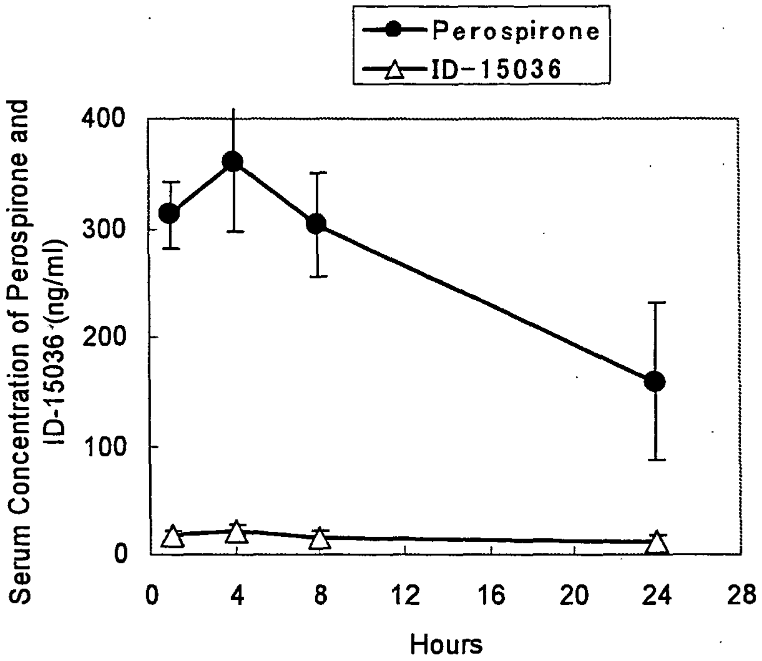


Figure 3

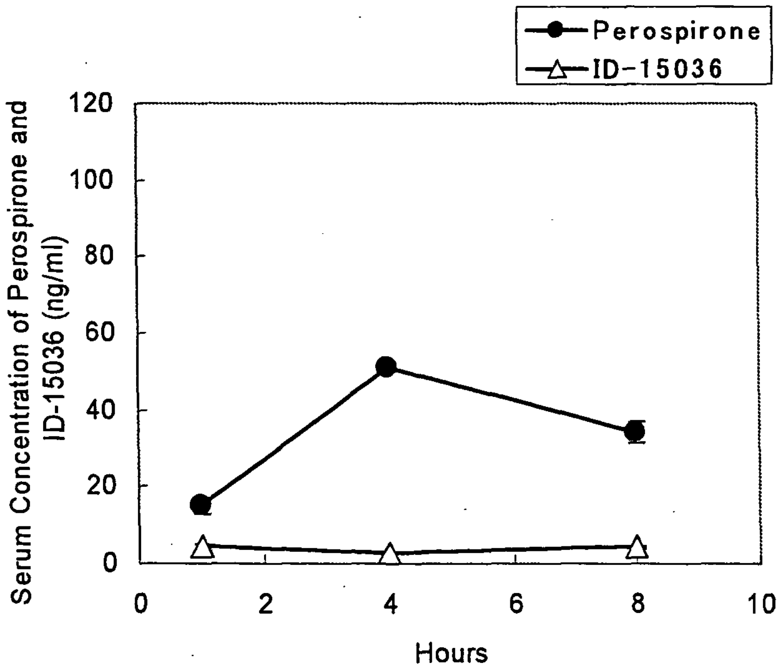

Figure 4

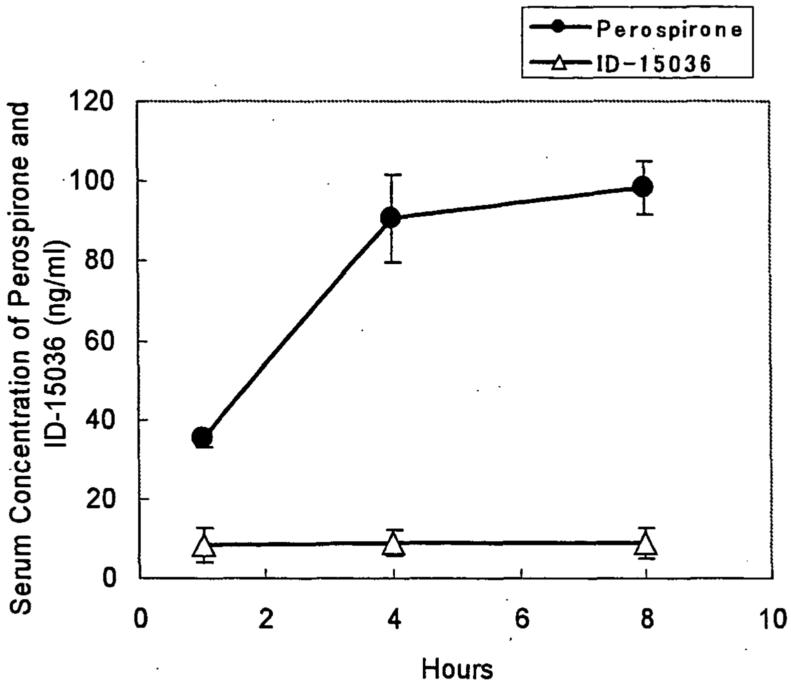


Figure 5

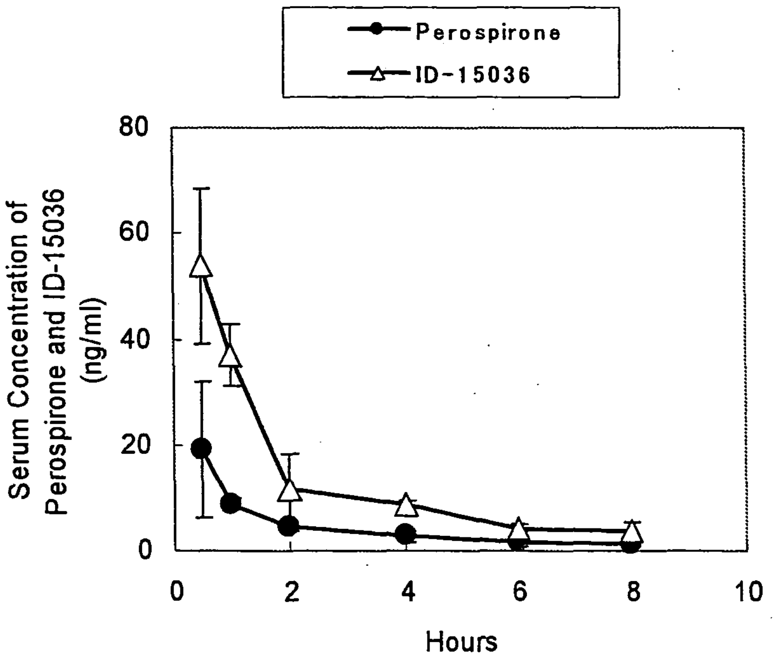

Figure 6

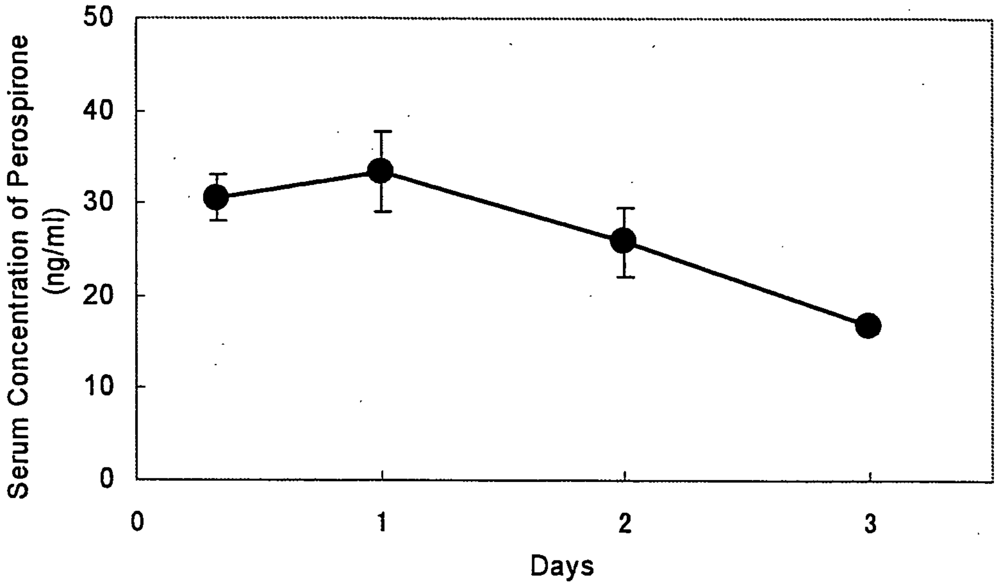
Serum Concentration of Perospirone (ng/ml)
50
40
30
20
10
0
0
1
2
3
Days

## INTERNATIONAL SEARCH REPORT

International application No.
PCT/JP2005/016084

A. CLASSIFICATION OF SUBJECT MATTER
Int.Cl[7] A61K31/496, 9/06, 9/08, 9/10, 9/70, A61P25/18, 43/00

According to International Patent Classification (IPC) or to both national classification and IPC

B. FIELDS SEARCHED

Minimum documentation searched (classification system followed by classification symbols)
Int.Cl[7] A61K31/496, 9/06, 9/08, 9/10, 9/70, A61P25/18, 43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAOLD(STN), CAplus(STN), REGISTRY(STN), JMEDPlus(JOIS), JST7580(JOIS), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| Y | Tadashi ISHIBASHI, "Ensan Perospirone Shuyo Taishabutsu no Chusu Yakuri Sayo", Kiso to Rinsho(1997), Vol.31, No.2, pages 893 to 902 | 1-8 |
| Y | Yukihiro OONO, "Tokushu Bio, I·Noyaku Kanren Shinki Koseishinbyoyaku Perospirone (Lullan) no Sosei to Kenkyu Kaihatsu", Sumitomo Kagaku (2001), Vol.2001, No.1, pages 38 to 45 | 1-8 |
| Y | JP 11-228414 A (SUMITOMO SEIYAKU KABUSHIKI KAISHA), 24 August, 1999 (24.08.99), (Family: none) | 1-8 |

☒ Further documents are listed in the continuation of Box C.    ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 21 September, 2005 (21.09.05) | 11 October, 2005 (11.10.05) |
| Name and mailing address of the ISA/ Japanese Patent Office | Authorized officer |
| Facsimile No. | Telephone No. |

Form PCT/ISA/210 (second sheet) (January 2004)

INTERNATIONAL SEARCH REPORT

International application No.
PCT/JP2005/016084

C (Continuation). DOCUMENTS CONSIDERED TO BE RELEVANT

| Category* | Citation of document, with indication, where appropriate, of the relevant passages | Relevant to claim No. |
|---|---|---|
| Y | JP 06-505712 A (SANO CORP.),<br>30 June, 1994 (30.06.94),<br>& WO 92/09252 A1 & AU 9191476 A<br>& EP 559825 A1 & EP 559825 B1<br>& DE 69127205 E & CA 2097182 C<br>& JP 2004-002465 A & US 5633009 A<br>& BR 1101015 A3 | 1-8 |

Form PCT/ISA/210 (continuation of second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 62123179 A
- JP 11228414 A
- JP 2651616 B
- WO 9612465 A
- JP 9278651 A


### Non-patent literature cited in the description


- *KISO TO RINSHO,* 1997, vol. 31, 543-568
- *KISO TO RINSHO,* 1997, vol. 31, 737-753
- *KISO TO RINSHO,* 1997, vol. 31, 2113-2157
- *KISO TO RINSHO,* 1997, vol. 31, 893-902
- *IYAKUHIN NO KAIHATU,* 1989, vol. 13, 87-133
- *American Journal of Industrial Medicine,* 1993, vol. 23, 711-719
- Keihi Tekiyou Seizai Kaihatu Manual. 1985
- *Jpn.J.Pharmacol.,* 1990, vol. 53, 321-329